Europäisches Patentamt

European Patent Office

Office européen des brevets

(11) Publication number: 0 348 042
A1

## (12) EUROPEAN PATENT APPLICATION

(21) Application number: 89305221.7

(51) Int. Cl.⁴: **A61K 35/74** , **C12N 5/00**

(22) Date of filing: 23.05.89

(30) Priority: 23.05.88 GB 8812129

(43) Date of publication of application:
27.12.89 Bulletin 89/52

(84) Designated Contracting States:
AT BE CH DE ES FR GB GR IT LI LU NL SE

(71) Applicant: **Wood, Christopher Robin**
**Gamekeepers Cottage Pinewood Road**
**Iver Heath Buckinghamshire SL0 0NJ(GB)**

Applicant: **Habib, Nagy Adly**
**8 Nichols Green**
**Ealing London W5 2QU(GB)**

(72) Inventor: **Wood, Christopher Robin**
**Gamekeepers Cottage Pinewood Road**
**Iver Heath Buckinghamshire SL0 0NJ(GB)**
Inventor: **Habib, Nagy Adly**
**8 Nichols Green**
**Ealing London W5 2QU(GB)**

(74) Representative: **Abrams, Michael John et al**
**HASELTINE LAKE & CO. Hazlitt House 28**
**Southampton Buildings Chancery Lane**
**London WC2A 1AT(GB)**

(54) **Method of modifying the lipid structure of cell membranes.**

(57) A method of modifying the lipid structure of cell membranes is disclosed which utilises the supernatant derived from a culture of Micrococcus cryophilus (ATCC 15174) or an extract therefrom. This method has utility in veterinary and clinical treatment of conditions characterised by an increase in the saturation index. Compositions incorporating the active ingredient are also provided.

EP 0 348 042 A1

## METHOD OF MODIFYING THE LIPID STRUCTURE OF CELL MEMBRANES

This invention relates to the modification of the lipid structure of cell membranes. The method of the invention is expected to find application in methods of treatment, both veterinary and clinical, and such treatment, both veterinary and clinical, and such applications also fall within the scope of the invention.

There is a wide range of naturally occurring saturated and unsaturated fatty acids. These generally have an even number of carbon atoms in the chain. The C18 acids (for example stearic, oleic, linoleic and linolenic acids) are well known.

The core of the cell membrane is the lipid bi-layer which acts as a "skeleton" for protein receptors and antigenic sites. The relative physical consistency of the bi-layer, known as membrane fluidity, depends mainly on the fatty acid content of the constituent lipids. This is regulated largely by the relative amounts of saturated and unsaturated fatty acids.

Original findings from our laboratory show that the regulation of membrane fluidity is dependent particularly upon the maintenance of an optimal level of desaturation of stearic (CAT:0,mp 60°C) into oleic acid (CAT:1, mp 13.9°C). The enzyme responsible for regulating this desaturation process is delta-9 desaturase and we believe that this enzyme plays a major role in membrane fluidity.

In our studies, we have adopted the ratio of stearic/oleic acids as a saturation index (SI) because, although we have noted changes in several fatty acids, the only consistent changes occurred in the stearic/oleic acid fractions. A more rigorous definition of "saturation index" is given hereinafter.

Perusal of published data and our own studies during the last few years have shown that the SI is increased or decreased in various pathological states of the cell. For example, the saturation index is decreased (due to an increase in unsaturated oleic acid) in cells infected by fusogenic syncitia forming viruses, in all continuous in vitro cell lines established from human and animal tissues, in malignant cells and tissues obtained from man and animals, in most peripheral blood cells obtained from patients with cancer and AIDS and in freshly cultured cells treated with urine from patients with malignancies.

Thus a decrease in the saturation of the cell membrane is widespread in cancer and certain virus infections and is, we believe, a major factor in the cellular dysfunction associated with these conditions.

Both the physical stability of cell membranes and the regulation of a wide variety of metabolic processes, espeially those involving membrane-associated enzymes, are dependent on the regulation of cell membrane lipid composition (Sandermann, H, Jr., (1978) Biochim.Biophys. Acta, 515, 209-237). This regulation provides for the homeostasis of membrane fluidity, predominantly through the balance between constituent saturated and unsaturated fatty acids (Doi, O; Doi, F; Schroeder, F; Alberts, A W & Vagelos, P R (1978) Biochim. Biophys. Acta, 509, 239-250; and Quinn, P J (1983) Biochem. Soc. Trans. 11, 329-330). Analysis of cell membrance lipids shows that the most abundant species of fatty acids are the 18 carbon (C18) straight chain fatty acids of which the polyunsaturates (e.g. linoleic and linolenic) acids are derived from the diet. Further, the other main components of this family, octadecanoic acid (stearic acid) and cis-9-octadecenoic acid (oleic acid), differ widely in their melting points and so the relative proportions of these acids within the membrane is important in determining membrane fluidity. It is generally accepted that cell membrane fluidity is essential for homeostasis and depends on the relative content of the saturated and unsaturated fatty acids in the constituent phospholipids. In normal resting cells, the degree of cell membrane fluidity appears to be associated with the rate of cell division, higher fluidity reflecting a higher rate of division, and vice versa. The main site of fat metabolism in the whole organism is the liver where all dietary lipid and free fatty acids are sequestered and reprocessed. Thus, virtually no free fatty acids are released into the general circulation.

Fatty acids are synthesised by chain elongation to palmitic acid (C16:0) and then on to stearic acid (C18:0). The main enzyme which catalyses desaturation is the lipid dependent, three component enzyme delta-9 desaturase (Jeffcoat, R and James A T in New Comprehensive Biochemistry, publ. Elsevier, 1984, vol. 7 pp 85-112). Palmitic acid is desaturated to palmitoleic acid (C16:1) and stearic acid to oleic acid (C18:1). However, the enzyme has higher affinity for stearic acid. These four fatty acids make up 60-80% of all fatty acids extractable from cells. The other C18 fatty acids, linoleic (C18:2) and linolenic (C18:3) also found in cell membranes, are essential fatty acids. They are not synthesized by mammalian cells, but are important as prostaglandin precursors. Thus, the delta-9 desaturase has an intrinsic central role in maintaining endogenous membrane fluidity and is found to be under tight cellular control. Under normal circumstances the activity of this enzyme is regulated in accordance with requirements of the cell to produce more oleic acid in order to maintain a specific level of membrane fluidity.

Isoviscosity is the term used to denote the adapted modulation of membrane fluidity in response to changes in ambient temperature. In physical terms, isoviscosity. is related to changes in the melting points and packing density of the constituent lipids. In bacteria, isoviscose adaptation involves chain length modulation as well as control of desaturation of fatty acids. In poikilothermic unicellular parasites, the adaptive changes in membrane fluidity mainly affect the regulation of fatty acid desaturation. Currently, evidence is emerging that in homeothermic animals and animal cell cultures, the regulation of membrane fluidity may be limited to the regulation of a unique double bond. In a variety of pathological and physiological conditions, lipid analysis of the affected cells shows predominant and consistent changes in the ratio of the saturated stearic acid to unsaturated oleic acid. The change in this ratio expressed as the saturation index (SI) is associated with the cytopathic effect of virus infected and toxin treated cells.

The use of essential fatty acids in various clinical and veterinary treatments has been described in several published patent applications. For example, EP 0037175, EP 0071357, EP 0078434 and EP 0087864 (all in the name Efamol Ltd.) relate to the use of linolenic acid (LLA) and dihomo-gamma linolenic acid (GLLA). These fatty acids influence the prostaglandin cycle and are set forth as useful in the treatment of a number of disorders including multiple sclerosis, cancer, allergic and inflammatory disorders, disorders of lipid metabolism in which blood cholesterol levels are elevated, hypertension, mental illness, schizophrenia and depression. In treating all of these conditions, the active ingredients are said to be LLA and GLLA. Natural sources of GLLA are recommended for therapeutic use; these include oils derived from the seeds of Oenothera species (Evening Primrose) and Borago officinalis (Borage). These oils from Oenothera biennis typically contain about 10% oleic acid. The presence of oleic acid in these oils is merely incidental and is not taught as being of any beneficial effect.

GB 1506563 (John Williams) is also concerned with the therapeutic use of LLA and GLLA. These acids are said to be effective in treating neurological disorders and diseases and act as immuno-suppressive agents.

GB 2134782 (Sentrachem Limited) discloses the use of LLA and GLLA in the treatment of cancer. It is claimed that the defect in cancer cells is an inhibition of the enzyme delta-6 desaturase which results in a block in the conversion of LLA to GLLA. The invention disclosed in this document involves the administration of GLLA, arachidonic acid, eicosapentaenic acid or their derivatives. There is no disclosure of any therapeutic effect associated with oleic acid or its unsaturated homologues.

US 4097602 (Melvin J Silver et al) discloses a method of inhibiting blood platelet aggregation by oral administration or parenteral administration of cis-8,11,14-eicosatrienoic acid which is said to influence the prostaglandin cycle. This polyunsaturated active ingredient may be combined with conventional compatible organic or inorganic pharmaceutical carriers.

GB 1440386 (Geraldine Hudson Thiele) discloses a process of accelerating the healing of a mosaic bone fracture by the use (as scleroting agents) of a variety of fatty acids. All saturated and unsaturated fatty acids, regardless of the chain length (and including formic acid) are claimed to be of use. Sodium oleate is exemplified in the treatment of metacarpal periostesis, ringbone and osselets in horses.

GB 1280244 (Parke Davis & Co.) discloses the use of alkanedioic acids and their salts and alcohol esters in compositions for reducing the level of triglycerides in serum. The disclosed active ingredients may be admixed with conventional pharmaceutical carriers.

GB 2104907 (Kureha Kagaka Kogyo KK) discloses the use of cyclodextrin as a stabilising reagent for eicosapentaenoic acid and docosahexaenoic acid, their salts and esters. The resulting inclusion compounds are said to reduce cholesterol levels in human serum.

GB 2090529 (Nippon Oil and Fats Co. Ltd.) discloses a thrombosis-preventing curative agent containing docosahexanoic acid or a derivative thereof. The active ingredient influences the prostaglandin cycle and antagonizes the action of arachidonic acid.

GB 2012162 (Duncan Lee McCollester) discloses an injectable composition containing a source of manganous ions for use in the immunotherapy of neoplastic disease. The manganous ion is said to stimulate the immune antigens and the nucleotide cyclase enzymes.

GB 1582992 (Proctor & Gamble) discloses the use of C4 -C12 carboxylic acid derivatives in a water soluble solution for intravenous administration in order to inhibit the growth of microbes. There is no discosure of the bacteriostatic and fungostatic properties of the active compounds.

Total lipid extracts of erythrocyte cell membranes from patients with documented malignancies, various acute and chronic diseases and healthy subjects have been analysed (British Medical Journal, 20 July 1985, 291, 163-165). . The results were expressed as ratios of stearic to oleic acid, reflecting the degree of desaturation of stearic acid in the cell membrances. This ratio of saturated C18 fatty acid to monounsaturated C18 fatty acid, when determined by the gas-liquid chromatographic techniques described in the above-mentioned article from total lipid extracts prepared according to the method described by Apostolov and Barker (FEBS Lett. 1981, 126, 261-264), is referred to herein as the "saturation index". The mean index for healthy subjects and controls without cancer were 1.5 (SD 0.27) and 1.45 (SD 0.28), respectively, whereas the index for patients with malignancies were consistently lower than 1.0 with a mean value of 0.69 (SD 0.15) (p less than 0.001).

WO87/04926 discloses that the parenteral administration of C12 - C28 mono-unsaturated fatty acids, e.g. oleic acid, in vivo, or the addition of these compounds to cell cultures in vitro, results in a modification of the saturation index in the cell membranes, and claims, for use in modifying the lipid structure of cell membranes, a mono-unsaturated fatty acid having from 12 to 28 carbon atoms in the alkyl chain, e.g. oleic acid, or a pharmaceutically acceptable derivative thereof, prepared for parenteral administration. Techniques for the treatment of various clinical conditions are also described in WO87/04926.

It is now well established that the properties of plasma membranes of transformed and malignant cells differ from those of normal cells (Spector and Burns, 1987). However, most of the investigations refer to the properties of the proteins in the membrane and their interaction with the lipid bilayer (Nicholson and Poste, 1976).

A general and consistent finding has been that in malignant and transformed cells, there is increased membrane fluidity. Three complementary sets of data support this characteristic of the malignant cell phenotype. First, fluorescent probes have demostrated increased membrane fluidity in leukaemic cells and in vesicles obtained from leukaemic cells (Van Bliiterswijk et al, 1977, Petitou et al, 1978). Second, NMR studies also showed increased membrane fluidity in tumour biopsy specimens (Mountford et al, 1986). There is also evidence that potentially malignant cells can be differentiated by this technique (Mountford et al, 1987). Third, comparative lipid analysis of malignant cells supports these findings and provides data which suggest an explanation (Apostolov et al, 1985, Wood et al, 1985). Analysis of the fatty acid content of a wide range of malignant and transformed cells from biopsy material and tissue culture have shown a higher content of oleic acid when compared to stearic acid (i.e. a lower SI) in relation to the control, normal cells from which they originated (Howard and Kritchersky, 1969, Maldonato and Bough, 1980, Wood et al, 1985, Habib et al, 1987, Worman et al, 1987).

A decrease in the saturation Index has also been noted in association with the cytopathic effect produced by certain viruses. The fusogenic syncitia-forming viruses produce a significant decrease in SI characterised by a reversible promotion of stearic acid desaturation. We have found this in association with infection with Newcastel Disease Virus (NDV), herpes simplex type II, Epstein-Barr virus and the Human Immuno-deficiency virus (HIV), which is responsible for AIDS (Blenkharn and Apostolov, 1981, Barker and Apostolov, 1987, Apostolov et al, 1987).

The exact mechanism whereby viruses cause cell death is not known. Cell death in virus infected cells is preceded by a change in morphology known as a cytopathic effect (CPE). We have found that the CPE is associated with significant changes in the SI.

Viruses induce two kinds of cytopathic effect in vitro and in vivo: firstly, fusogenic syncitia-forming viruses produce a significant decrease in SI characterised by a reversible promotion of stearic acid desaturation; and secondly, a lytic effect characterised by host cell rounding and detachment which is associated with severe but reversible inhibition of stearic acid desaturation, resulting in an increase in the saturation index. This type of change has been demonstrated for the Syndbis, coxsackie and herpes simplex type I viruses.

There is also published evidence that interferon (IFN) increases cell membrane rigidity and also inhibits the release of budding enveloped virus particles.

In tissue culture when cells were exposed to IFN for one hour and then the IFN removed, there was an increase in the saturation index, reaching maximum values between 10-12 hours. This was followed by a rebound effect, a depression in the SI below normal cell values and a return to normal within 72 hours. Intermittent replenishment of the overlay of IFN treated cells with fresh medium led to a progressive increase in the SI and the appearance of a cytopathic effect. The overlay medium of cells post treatment with IFN, when purified and concentrated, contained a protein which was antagonistic to IFN and its effects on the SI.

The use of interferon in the treatment of malignancies follows from the demonstration of its capacity to inhibit cell division. We have shown that IFN treatment of cells in vitro induces a reversible increase in the SI and we propose that the main biological effects of IFN are due to this effect on in the SI. Support for the concept also comes from the finding that IFN treatment of patients with hairy cell leukaemia is associated with an increase in the abnormally low SI in peripheral blood cells.

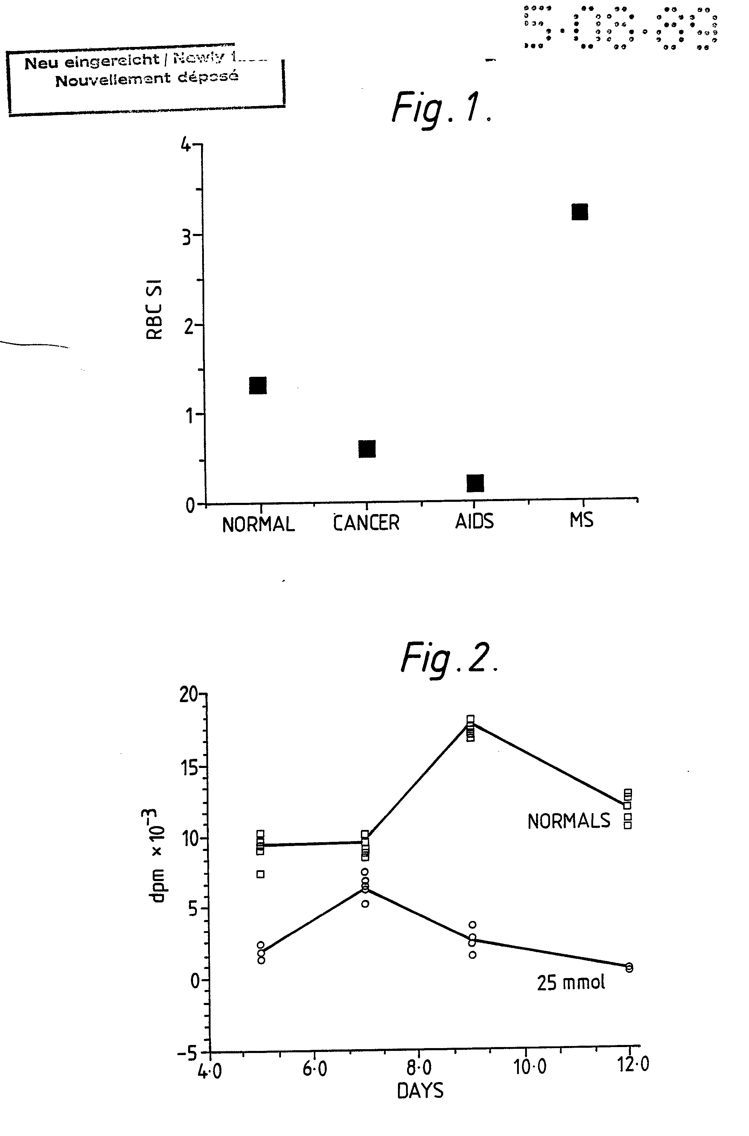

We have studied the red blood cells saturation index in a group of patients with various neurological conditions including multiple sclerosis (MS), schizophrenia, Alzheimers disease and also a small number of patients with chronic alcoholism. Sixteen patients with multiple sclerosis were studied initially and the mean RBC saturation idex for this group was 3.26 (range 1.8 -7.6). This elevated saturation index was significantly higher than the normal control population. Figure 1 illustrates the mean SI values of these MS patients and of typical groups of patients with cancer and AIDS.

A similar rise in the saturation index of the red blood cells was found in a group of patients with schizophrenia, and also some patients with chronic alcoholism.

This rise in the saturation index of the red cell membranes is due to a large increase in the stearic acid content, suggesting inhibition of the delta-9 desaturase enzyme system.

There is some evidence to suggest a derangement of lipid metabolism in both multiple sclerosis and schizophrenia. The pathological feature of multiple sclerosis is the demyelimination of the neural sheath and the deposition of fatty plaques along the nerves. Furthermore, improvement in clinical status has been noted in MS patients given diets rich in polyunsaturated fats, although conflicting data has also been published.

Some interesting work has been done by Crowe and his colleagues, on the cytopathic effect of cerebro-spinal fluid (CSF) from patients with schizophrenia. They noted that a substantial proportion of cerebro-spinal fluid (CSF) samples taken from schizophrenics and other neuro-psychiatric cases induced a cytopathic effect in cell culture (Tyrrell et al, 1979). The cytopathic effect (CPE) was seen in CSFs from a variety of neuro-psychiatric conditions (Crowe et al, 1979), but not in non-neurological control CSF. They postulated at first that a virus was responsible for the CPE, but later work showed this was unlikely. It is tempting to postulate that the causative factor for these neuro-psychiatric conditions induces the change we have noted in the serum and is the factor isolated by Crowe et al in CSF.

Another area where membrane fluidity is of considerable significance is ageing. The central concept of ageing is loss of adaptability of an individual organism with time, so that, in general, the old are more vulnerable to environment challenge than the young and response to thermal or other stresses is less with increasing age. It is apparant clinically that fatality of disease, including trauma, surgical operations and infections, is increased in the elderly. In general, functions requiring integrated actions of several body systems show greater decline than functions of single systems. Homeostatic mechanisms tend to become slower, less sensitive and less accurate with age.

In order to understand the various paterns associated with old age, it is important to investigate the changes occurring in the ageing cells in vitro. It is also relevant to study the changes that occur in transformed or malignant cells as their behaviour is just the opposite to that of the ageing cells.

Although cells derived from malignant tissue or those which have undergone viral transformation, or chromosomal aberration, may produce immortal cell lines in tissue culture, clones or normal differentiated cells die out after a limited number of population doublings and death is preceded by a phase of reduced or disordered function.

In contrast to the malignant cell, aged cells lose their ability to divide. Therefore we have speculated that ageing cells would have a higher saturation index than malignant or normal "younger" cells.

In order to test this theory we studied the saturation index of a variety of cell lines. Table 1 shows the mean (+ SEM) saturation index of human embryonic lung cells grown to 50% confluence. Triplicate samples were taken each day and the fatty acid components of the cell membranes were estimated. .The results show a progressive rise in the saturation index from $1.02 \pm 0.11$ on the first day of sampling to $5.30 \pm 0.41$ at 7 days:

TABLE 1

| DAY | SATURATION INDEX (SI) | | | MEAN SI (+SEM) |
|---|---|---|---|---|
| 0 | 0.87 | 1.23 | 0.96 | 1.02±0.11 |
| 1 | 1.25 | 1.38 | 1.62 | 1.42±0.11 |
| 2 | 1.79 | 2.40 | 1.85 | 2.01±0.19 |
| 3 | 3.72 | 3.41 | 2.86 | 3.33±0.25 |
| 4 | 2.95 | 3.06 | 2.87 | 2.96±0.06 |
| 5 | 5.61 | 4.23 | 4.76 | 4.87±0.40 |
| 6 | 4.44 | 5.92 | 5.55 | 5.30±0.44 |

Thereafter the cells had shattered and no more cells could be recovered from the culture.

Similar results were obtained for primary bovine kidney (PBK) cells grown in culture. The results of this study are shown in Table 2 and once again show a progressive rise in the mean saturation index from day 4 (saturation index = $1.10 \pm 0.14$) to day 32 (SI = $3.98 \pm 0.26$):

TABLE 2

| DAY | SATURATION INDEX (SI) | | | MEAN SI (+SEM) |
|---|---|---|---|---|
| 4 | 1.15 | 0.83 | 1.32 | 1.10±0.14 |
| 8 | 1.16 | 1.33 | 1.42 | 1.30±0.10 |
| 12 | 0.92 | 1.58 | 1.16 | 1.22±0.19 |
| 16 | 1.36 | 1.59 | 1.43 | 1.46±0.07 |
| 20 | 1.48 | 1.52 | 1.72 | 1.57±0.07 |
| 24 | 1.62 | 1.59 | 1.92 | 1.71±0.10 |
| 28 | 2.36 | 2.76 | 2.86 | 2.65±0.15 |
| 32 | 4.48 | 3.62 | 3.85 | 3.98±0.26 |

Thus, in vitro, there appears to be an increase with time in the saturated fatty acid content of cell membranes which appears to be related to the ageing process of the cell.

Further evidence was found in our studies of rat blood samples. The red blood cell saturation index was measured in rats of various ages. Table 3 shows the principal fatty acid components of the cell membranes in animals aged 1-10 weeks, 11-20 weeks and 21-30 weeks expressed as a percentage of the total fatty acids present. The major change occurs in the stearic acid fraction with an increase in this fraction from 32.4 ± 4.9 in the younger age group to 48.4 ± 9.8 in the older rats. Relatively little change occurred in the oleic acid fraction although some decrease was seen with time. When the saturation index was measured, this represented a significant increase in the index in the ageing rats (p≤ 0.02):

TABLE 3

| AGE, WEEKS | 1-10 | | 11-20 | | 21-30 |
|---|---|---|---|---|---|
| Palmitic | 33.3±6.3 | | 33.5±5.5 | | 30.9±5.7 |
| Palmitoleic | 1.3±1.0 | | 0.5± 0.8 | | 0.5±0.9 |
| Stearic | 32.4±4.9 | | 40.4±10.4 | | 48.4±9.8 |
| Oleic | 16.2±1.5 | | 14.5± 2.7 | | 13.2±2.8 |
| Linoleic | 13.6±3.7 | | 10.2± 4.3 | | 6.1±3.1 |
| Linolenic | 2.6±5.1 | | 0.9± 1.3 | | 0.8±0.8 |
| SI | 2.0±0.4 | | 3.1± 1.9 | | 1.0±0.3 |
| | | <0.04 | | <0.02 | |

The effect of adding exogenous stearic acid to cell culture can be demonstrated from our studies of epidermal cells from normal individuals. Figure 2 shows the counts per minute after the administration of tritiated thymidine to the cultured cells.

There is a rise in tritiated thymidine counts up to approximately 10 days of the culture, after which the cells begin to die and this is associated with a drop in DNA synthesis. Figure 2 also shows the same cells with the addition of stearic acid to the culture medium. DNA synthesis is less than in the normal cells, reaching a much lower peak at approximately 7 days, and death of the cells appears to occur earlier than the normal. This information may be of relevance to the cosmetic industry where stearic acid is used as a base for ointments and creams etc.

Some interesting work on the effect of exogenous fatty acids has been performed by Shih and Young (1978). They noted that the number of serotonin binding sites in brains from old men is about 2-fold higher than in brains from young adults. The over exposed receptors can overshoot the point of optimal activity and eventually become more vulnerable to degradation. These authors noted that some of the behavioural aspects of ageing, associated with the serotoninergic system (e.g. loss of sleep, appetite and libido) could be accounted for by this process.

The accessability of a receptor binding site, which is a prerequisite for its function, and the extent and direction of its displacement by changes in the lipid fluidity, are determined by the geometry of the receptor in the immediate lipid environment. Heron et al (1980) studied the passive modulation of the accessability of the serotonin receptor in brain membranes and found an increase in the microviscosity of the synoptic

6

membranes by in vitro incubation with stearic acid. This resulted in an up to 5-fold increase in the specific binding of tritiated serotonin. Fluidization of membrane lipids by treatment with lecithin or linoleic acid caused a small but significant decrease in serotonin binding. The increased fluidity of the membrane is even greater with oleic acid. Thus, we postulate that the ability to prevent or reduce the increased saturation could be playing a major role in cellular ageing.

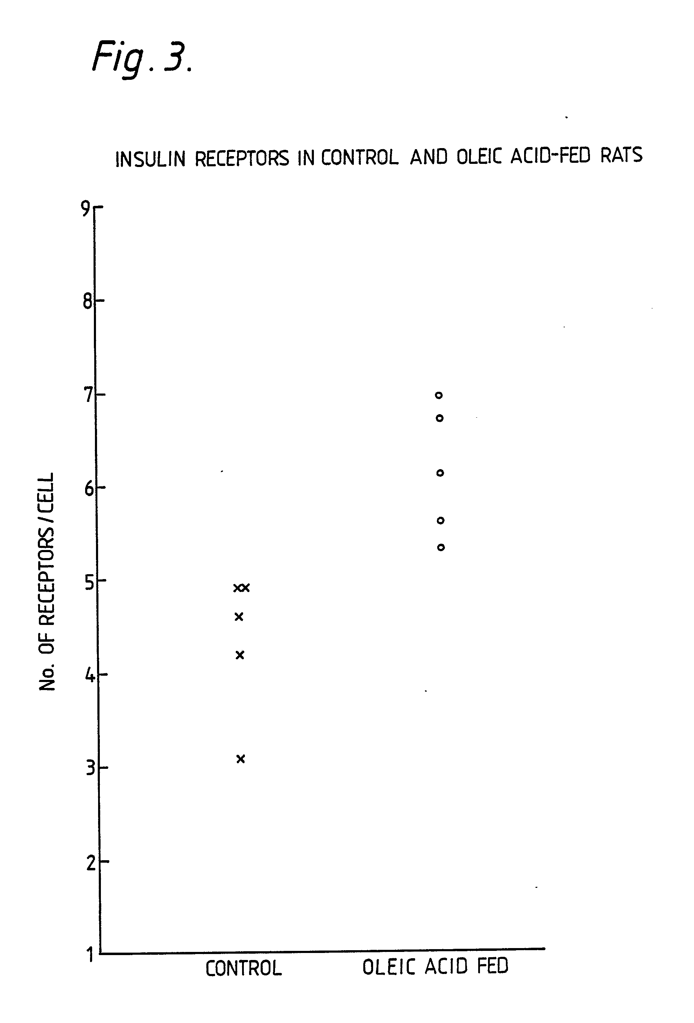

The use of oleic acid as a means of inducing desaturation within the body cells is fraught with difficulty. Given by the oral route, it will rapidly be processed within the intestine and the liver and little or none will reach the cells in active form. However, an experiment that we have performed suggests that prolonged dietary intake of oleic acid in rats can lead to profound changes in cell membrane receptors. In this experiment rats were fed from an early age on either a control normal diet or a diet rich in oleic acid. After nine months the animals were sacrificed and organs analysed for saturation index of the membrane and also for insulin receptor status. Figure 3 shows that in the oleic acid fed animals there is a significant increase in the number of insulin receptors on the brain cells at the time of sacrifice. These cells would then be more receptive to insulin and to its pharmacological and growth properties. Further difficulties are encountered when giving oleic acid by the intravenous route, not least of which are the potential for fat embolus, local tissue reaction and poor patient compliance.

We have now investigated the ability of other substances to induce desaturation. One of the most interesting is a compound related to an organism Micrococcus cryophilus (ATCC 15174). This organism has the unique property of having a desaturated cell membrane at normal temperature. It is capable of changing its membrane fluidity with alterations in the ambient temperature. Accordingly, the present invention provides a method of modifying the lipid structure of cell membranes in vitro and in vivo, which comprises treating the cells with supernatant derived from a culture of Micrococcus cryophilus (ATCC 15174).

In our studies, we grew the micrococcus at room temperature, 4°C and 37°C and applied the supernatant after seven days culture to a human colon cancer cell line. After 24 hours the cells were harvested and the fatty acid components of the cell membranes measured. The results are shown in Table 4 and clearly there has been desaturation of the cell membranes treated with supernatant from the micrococcus grown at room temperature and 4°C. No change was noticed in the supernatant from micrococcus grown at 37°C. However, if the micrococcus pellet was sonicated and then the supernatant added again to the cells, the desaturation was noted at the three temperature points.

TABLE 4

| (A) SUPERNATANT ALONE | |
| --- | --- |
| TEMPERATURE | S.I |
| R.T | 0.566 |
| 4°C | 0.577 |
| 37°C | 0.835 |
| Control | 0.725 |
| (B) SUPERNATANT FROM SONICATED PELLET | |
| R.T | 0.391 |
| 4°C | 0.556 |
| 37°C | 0.469 |

Similarly application of the supernatant from the micrococcus after seven days' growth to primary bovine kidney cells in culture showed a similar fall in the saturation index of the cells. The SI at room temperature after 24 hours, incubation with the micrococcal supernatant was 0.683, at 4°C was 0.489 and at 37°C was 0.562. This was compared with the control saturation index of these cells of 1.08.

Further work has been done with the application of the micrococcus supernatant to cultured cervical cancer cells and also normal epidermal skin cells. The cervical cancer cells are an established cell line derived from a patient with cervical cancer. These cells are particularly responsive to epidermal growth factor and their characteristics have been well defined. Application of a micrococcus supernatant to these cells leads to a decrease in saturation index, an increase in cell number and uptake of tritiated thymidine compared with untreated cells. These data indicate that the application of the micrococcus supernatant is capable of stimulating cell growth and DNA synthesis.

Similarly, when the supernatant was applied to epidermal cells in culture a similar result was obtained. These epidermal cells were obtained from a healthy patient undergoing a routine operation. The cells were grown in culture for seven days before sub-culturing and the establishment of a continuous cell line. As with the tumour cells, the application of the micrococcus supernatant led to a decrease in the saturation index of these cells, an increase in cell number and an increase in tritiated thymidine uptake.

An associated feature of these experiments was that when the micrococcus supernatant was added to the cells causing increased cell turnover, there was also a significant increase in the number of insulin receptors on the individual cells. This demonstrates that the growth factor contained in the micrococcus supernatant is capable of altering the cell membranes and the state of the receptor proteins within the membrane. This may have important implications for treatment of a variety of disease processes.

The ability of the micrococcus supernatant to sustain an increase in the growth of cells was also demostrated in a series of experiments with peripheral blood cells and bone marrow stem cells. Plasma cells were obtained from a patient with multiple myeloma and transferred to the supernatant from the micrococcus culture. In a similar way, bone marrow stem cells were obtained from a marrow aspirate from a normal individual and placed in the supernatant. It should be noted that the supernatant was not diluted nor concentrated nor was the medium changed during a 14-day period. However, the plasma cells and the bone marrow stem cells remained alive and apparently healthy over 14 days. The experiment was then stopped but we anticipate that it should be possible to maintain these cells in culture for a much longer period of time since the cells showed no evidence of dying.

We have attempted to define the active ingredient within the supernatant. Electrophoresis has demonstrated several protien bands and a wide range of lipids. It should be noted that the saturation index of the micrococcus is very low at 0.2 which is in keeping with the published literature on the organism, i.e. that it is permanently desaturated at low temperature. Our experiments show that the protein fractions do not seem to have a desaturating effect on cell membranes although our experiments are not complete and the active ingredient may well be in this fraction. When the lipids were extracted from the supernatant they had a profound desaturating effect on cultured cells (see Table 5). This strongly suggests that the active ingredient, the desaturating factor, is in a lipid fraction.

We think the ability to desaturate cell membranes and in particular, the use of the active ingredient within the micrococcus supernatant has potential in a number of areas in accordance with this invention, including the following:-

i. In diseases in which the cells, or some of the cells in the body, have a higher proportion of saturated fats relative to unsaturated fats as compared to normal cells. These conditions include multiple sclerosis, schizophrenia, some forms of chronic alcoholism and some patients with hypercholesterolaemia. This list is not meant to be exclusive and there may be other conditions which are characterised by a higher than normal saturation index or an increase in unsaturated fat within the cell membrane.

ii. To alter cell membrane receptor function by changing the lipid components of the membrane. This may be important in conditions such as insulin dependent diabetes by changing the lipid component of the cell membrane in order to increase the number of insulin receptors.

iii. To alter the lipid component of the cell membrane to prevent abnormal uptake or deposition of harmful lipids. This may be important in conditions such as hypercholesterolaemia and atherosclerosis.

iv. To stimulate cells to prevent the normal ageing process. This is particularly relevant to skin cells and our work has demonstrated that skin cells can be stimulated to divide when the saturation index is lowered. A desaturating factor such as that found in the supernatant of the micrococcus growth medium could have applications in a number of areas including the pharmaceutical industry as treatment for various neurological conditions, hypercholesterolaemia, atherosclerosis and diabetes mellitus. Also an application exists in the medical and biological field with the ability to grow and maintain cells in culture. The third area in which this ability to desaturate and in particular, the use of the micrococcus supernatant growth factor is in the cosmetic industry with the possible potential for an anti-ageing substance.

The micrococcus supernatant described above has been tested for cytotoxic and cytostimulatory properties in vitro, using the MTT assay. For convenience, the undiluted material is referred to hereinafter as "Compound 509". The effect of Compound 509 on two cell lines - namely RT112 - Bladder tumour - and HU609 -Normal urothelium - was investigated using the MTT assay. This is described by Mosmann (1983) and is the assay of choice of the National Cancer Institute for drug screening, who test as many as 10,000 compounds a year on 100 cell lines. The colorimetric assay is based on the ability of live but not dead cells to reduce a soluble tetrazolium based compound, 3,4,5-dimethylthiazol-2,5-diphenyl tetrazolium bromide (MTT) to a blue insoluble formazan product (Salter et al 1963). The results of this assay give a total cell count, whereas the clonogenic assay determines the proportion of cells able to form colonies of 50 or more cells, hence the MTT assay can determine stimulatory effects as well as inhibitory.

The results observed were that, with RT112 at 100 microgram/ml, compound 509 shows no effect upon the tumour cells. With HU609, at 100 microgram/ml, compound 509 shows stimulatory effects upon the normal cells (49-66%). At 150 microgram/ml 84% stimulation is seen.

The effects of compound 509 on the SI of cells two cells live HU609 was also investigated. The results are given in Table 5 below:

TABLE 5

|  | 10 · hours | SI after | 3 days |
|---|---|---|---|
| Control | 1.41 | | 1.07 |
| 20 microgram/litre compound 509 | 1.67 | | 1.16 |
| 300 microgram/litre compound 509 | 1.07 | | 0.82 |

The fatty acid composition of compound 509 was investigated. The results are set out in Table 6 below:

## TABLE 6

### Fatty acids composition of 50g

### $\underline{x \quad + \quad SE}$

| | | |
|---|---|---|
| Palmitic | 14.8 $\pm$ | 9.9 |
| Palmitoleic | 13.6 $\pm$ | 5.2 |
| Stearic | 5.7 $\pm$ | 5.7 |
| Oleic | 28.6 $\pm$ | 6.7 |
| Linoleic | 1.4 $\pm$ | 1.3 |
| Linolenic | 1.4 $\pm$ | 1.4 |
| Arachidonic | 0 | |
| $E_i$cos. + Arich | 0.15 $\pm$ | 0.15 |
| Others | 28.2 $\pm$ | 1.91 |

An analysis of the of micrococcus supernatant (compound 509) will now be described.

Analysis by polyacrylamide gel electrophoresis in SDS

Samples were run in 5% polyacrylamide gels on a flat-bed system using an LKB Multiphor 2117 apparatus. Sample A was the supernatant after growth of micrococcus cryophilus (ATCC 15174), as described above, while sample B was a control (the growth medium in the absence of the micrococcus).

Each sample was run with and without reduction (0.2M-mercaptoethanol for 2min at 95°C.

Staining for proteins and carbohydrates was carried out:

1. Proteins were fixed in glacial acetic acid: methanol and stained with Coomassie Blue.

2. Carbohydrate was fixed in isopropyl alcohol:acetic acid and stained using the periodate/silver method. Both were destained with acetic acid.

Standards of normal human serum and ferritin were run giving molecular weight markers from 400kDa (ferritin) to 69kDa (albumin) and below.

RESULTS

Only sample A gave significant staining. A range of protein bands were visible between approx. 200kDa and 20kDa some of these bands stained for carbohydrate.

Sample A also contained three very high molecular weight bands staining only for carbohydrate. These bands were very sharp and were all above 500kDa. The two highest bands were close together with an estimated molecular weight >900kDa.

FRACTIONATION OF SAMPLE A

Samples of Fraction A were noted to be cloudy and to contain some material which sedimented on standing. The complete fraction and that centrifuged at 5000g for 10min were compared by fractionation on Sepharose CL 4B.

Samples were separated on columns of Sepharose CL 4B. This gel filtration medium gives fractionation of proteins in the range 80,000 to 20 million molecular weight and for polysaccharides in the range 30,000 to 5 million.

The columns were either 30cm x 1cm or 30cm x3cm (see below). Initial experiments were carried out to evaluate the separation of the very high molecular weight material (carbohydrate rich) from the lower molecular weight, mainly protein containing fraction seen on electrophoresis. Columns were equilibrated and run in 0.2-M sodium phosphate buffer pH 7.8.

Samples applied to the column without centrifugation left a sediment on the top of the column and also had an excluded peak of carbohydrate and 280nm absorbing material. The main peak, included on the column contained both carbohydrate and 280nnm absorbing material.

Centrifuged samples contained no excluded peak and separated into two fractions, a minor fraction of higher molecular weight and a main fraction. Both fractions contained carbohydrate and 280nm absorbing material.

Separation of the centrifuged material was scaled up using a larger sized column of Sepharose CL 4B (210 ml, 30cm x 3cm) in order to generate larger batches of the two fractions for analysis

Yield of fractions from 10ml of centrifuged sample A:

FRACTION 1 - 4mg (400mg/1)

FRACTION 2 - 35mg (3.5g/1)

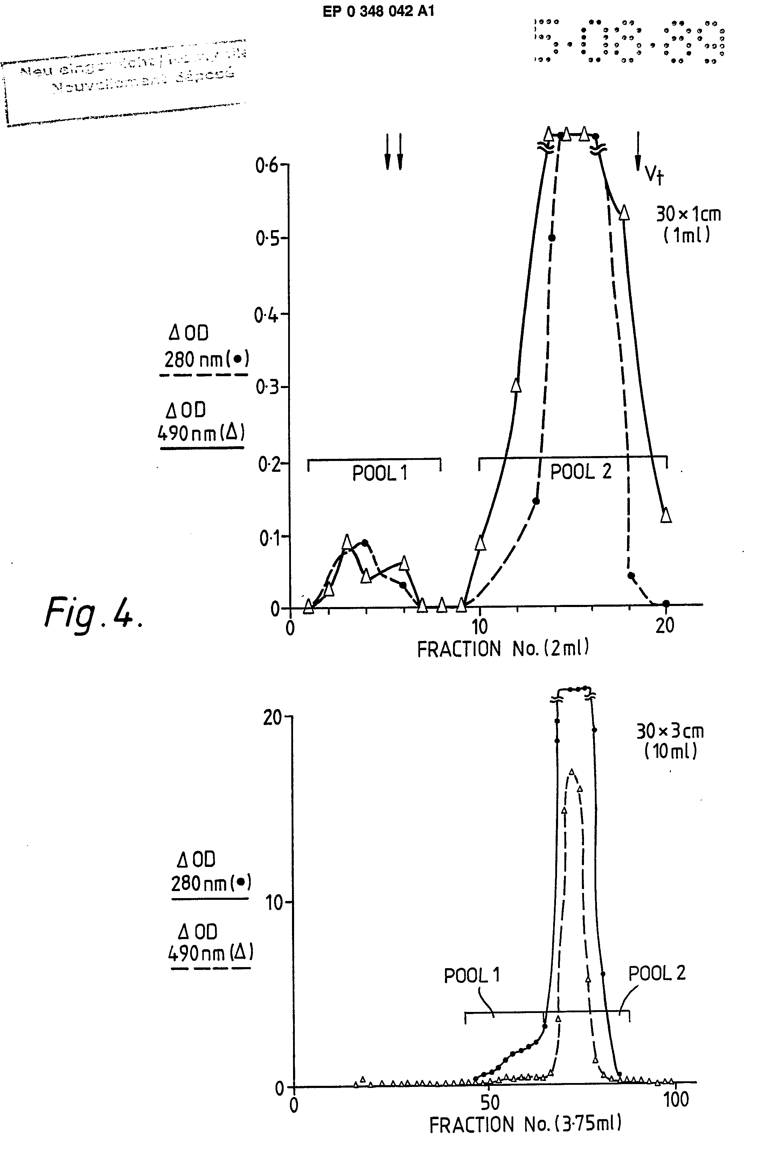

Details of these separations are shown in Fig 4.

Sample A was run on Sepharose CL 4B with and without centrifugation. 1. No centrifugation, column size 30cm x 1cm. 2. centrifugation on column the same size and 30cm x 3cm. Pools 1 & 2 are indicated. The limits of the column are shown as Vo and Vt.

Membrane fluidity refers to the physical state of the fatty acyl chains of the membrane lipid bilayer. Differences in the length of the hydrocarbon chain and degree of desaturation of the fatty acids play an important role in the determination of the fluidity of the cell membrane (Stubbs and Smith 1984).

Saturated and unsaturated fatty acids exhibit different structural conformations. The hydrocarbon chains of saturated fatty acids are flexible and can exist in a large number of different conformations, the most favourable being fully extended as this required minimal energy. Each double bond in unsaturated fatty acids forms a rigid kink in the hydrocarbon chain due to the inability of the double bond to rotate. These "rigid kinks" make it more difficult for the hydrocarbon chains to pack together in the cell membrane and hence the membrane is said to show greater fluidity.

There are reports of lipid modification of the cell membrane altering its fluidity and affecting cellular functions, including certain membrane bound enzymes and cell growth (Spector and Yorek 1985).

METHOD

Cell line: RT112

1) Trypsinize sub-confluent cells from 3 petri dishes (100 x 15).

2) Centrifuge 1000rpm, l0mins and resuspend in sucrose buffer.

Sucrose buffer: 10mM Tris HCl

0.25M sucrose 1.5mM glutathione

3) Sonicate.

4) Centrifuge 7000g 15mins
5) Centrifuge supernatant 100,000g 1hour
6) Resuspend pellet in 2.5ml 10mM $PO_4$ buffer
7) Read O.D. 500nm using U.V. spectrophotometer. (If necessary, store 70°C).
8) Suspension labelled with diphenylhexatriene (DPH) for 1 hour at 37°C.
9) Fluorescence measured with an analogue T format fluorimeter.
The results of this test with RT112 cells in the presence of compound 509 are shown in Table 7 below:

## TABLE 7

## Effect of 509 on membrane

## Fluidity of RT112 cell line

| | Mean Polarisation |
|---|---|
| control | 0.242 |
| 509 Treated cells 500micrograms/ml | 0.233 |

## Claims

1. A method of modifying the lipid structure of cell membranes in *vitro* and/or in *vivo*, which comprises treating the cells with supernatant derived from a culture of Micrococcus cryophilus.

2. A method according to claim 1, wherein a high molecular weight lipid fraction of said supernatant is used.

3. A pharmaceutical composition comprising supernatant derived from a culture of Micrococcus cryophilus or an extract therefrom.

4. A cosmetic composition comprising the supernatant derived from a culture of Micrococcus cryophilus or an extract therefrom.

5. A method of treating veterinary or clinical disorders characterised by an elevated saturation index (as hereinbefore defined), which comprises administering supernatant derived from a culture of Micrococcus cryophilus or an extract therefrom.

## Fig.1.

## Fig.2.

# *Fig. 3.*

INSULIN RECEPTORS IN CONTROL AND OLEIC ACID-FED RATS

Fig.4.

European Patent Office

**PARTIAL EUROPEAN SEARCH REPORT**
which under Rule 45 of the European Patent Convention
shall be considered, for the purposes of subsequent
proceedings, as the European search report

Application number

EP 89 30 5221

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | WO-A-87 04 926 (HABIB) <br><br> * Whole document * <br><br> -- | 1-4 | A 61 K 35/74 <br> C 12 N 5/00 |
| A | CHEMICAL ABSTRACTS, vol. 88, 1978, page 261, abstract 47315p, Columbus, Ohio, US; N.J. RUSSEL: "Desaturation of fatty acids by the psychrophillic bacterium Micrococcus cryophilus" & BIOCHEM. SOC. TRANS. 1977, 5(5), 1492-4 <br><br> * Whole abstract * <br><br> -- | 1-4 | |
| A | CHEMICAL ABSTRACTS, vol. 104, 1986, page 331, abstract 221808k; Columbus, Ohio, US; G.M. LLOYD et al.: "The translocation of phospholipids and wax esters from the cytoplasmic to outer membrane of the cell envelope in Micrococcus cryophilus" ./. | | TECHNICAL FIELDS SEARCHED (Int. Cl.4) <br><br> A 61 K <br> C 12 N |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 3,4
Claims searched incompletely: 1,2
Claims not searched: 5
Reason for the limitation of the search:

Claims 1,2: Searched in as far as the method relates to an "in vitro" practice of such method
Claims 1,2,5:
Method for treatment of the human or animal body by surgery or therapy (see art. 52(4) of the European Patent Convention).

| Place of search | Date of completion of the search | Examiner |
|---|---|---|
| The Hague | 01-09-1989 | MADDOX |

EPO Form 1505.1 03.82

| Category | Citation of document with indication, where appropriate, of relevant passages | Relevant to claim |
|---|---|---|
| | **DOCUMENTS CONSIDERED TO BE RELEVANT** | |
| | & BIOCHEM. SOC. TRANS. 1986, 14(2), 302-3 | |
| | * Whole abstract * | 1-4 |
| | -- | |
| A | CHEMICAL ABSTRACTS, vol. 108, 1988, page 395, abstract 62240k, Columbus, Ohio, US; "Extra pure unsaturated fatty acids" & PARFUME COSMET., AROMES 1987, 75, 79-80 | |
| | * Whole abstract * | 1-4 |
| | -- | |
| A | DE-A-1 903 335 (KYOWA HAKKO KOGYO) | |
| | * Example 6; claims 3,8 * | 1-4 |
| | -- | |
| A | EP-A-0 066 284 (AJINOMOTO) | |
| | * Page 5, lines 8-14 * | 1-4 |
| | -- | |
| A | WO-A-84 03 710 (INSERM) | |
| | ------- | |

**CLASSIFICATION OF THE APPLICATION (Int. Cl.4)**

**TECHNICAL FIELDS SEARCHED (Int Cl.4)**